# EUROPEAN PATENT APPLICATION

(11) **EP 3 876 081 A1**
(43) Date of publication of application: **08.09.2021**
(21) Application number: 19878317.7
(22) Date of filing: 30.09.2019
(51) Int. Cl.: G06F 3/01, G06F 3/16, G06T 7/00, G06T 7/20, G10L 15/00, G10L 15/22

(54) **CONFERENCE SUPPORT SYSTEM**

(30) Priority: 01.11.2018 JP 2018206524
(71) Applicant: Shin Nippon Biomedical Laboratories, Ltd., Kagoshima 891-1394 (JP)
(72) Inventor: NAGATA Ryoichi, Kagoshima-shi, Kagoshima 891-1394 (JP)
(74) Representative: Pitchford, James Edward
(86) International application number: PCT/JP2019/038647
(87) International publication number: WO 2020/090324

(57) **Abstract**

[Problem] To enable a person intended by a chairperson to be effectively determined as a speaker by matching the hand-raising of the speaker and an indicating direction of the chairperson, and turning ON only a microphone of the matched speaker. [Solution] Provided is a conference support system including: a plurality of voice input means which receive voices as an input; a plurality of ON/OFF control means which control the plurality of voice input means to be respectively turned ON/OFF and which respectively correspond to the plurality of voice input means; a hand-raising recognition means which recognizes hand-raising positions of participants, which respectively corresponds to the plurality of voice input means; a designated direction recognition means which recognizes a direction designated by a specified person; and a speaker determination means which turns ON an ON/OFF control means for a voice input means that corresponds to a participant, when the hand-raising position of the participant, which is recognized by the hand-raising recognition means, matches the direction designated by the specified person, which is recognized by the designated direction recognition means.

## Description

### TECHNICAL FIELD

The present invention relates to a conference support system.

### BACKGROUND ART

JP-A-7-15711 discloses a speaker automatic photographing device. This device determines a voice signal at a highest input signal level among voice signals input to voice input means, such as a microphone, as a speaker.

Patent Document 1: JP-A-7-15711

### DISCLOSURE OF THE INVENTION

### PROBLEMS TO BE SOLVED BY THE INVENTION

The conventional conference support system is basically a system that determines a person who emits the largest voice to a microphone as a speaker. This system has a problem that a person who significantly hinders the progress of the conference including a person making a loud roar (heckling) is determined as a speaker, thus interfering with the conference against the intention of a moderator. For example, there is a problem that, in a meeting, such as a stockholder meeting, since what is called a corporate extortionist or an angry stockholder shouts, employing the conventional conference support system causes these persons to be determined as the speakers, thus disturbing the stockholder meeting.

### SOLUTIONS TO THE PROBLEMS

One of a plurality of embodiments of this Description is based on a knowledge that a person intended by a moderator can be effectively determined as a speaker by matching a raise-hand of a speaker with an indicating direction of the moderator and turning ON only a microphone of the matched speaker.

The system includes a plurality of microphones for conference participants.

Then, in the system, the plurality of microphones can be individually controlled to be turned ON/OFF (powered on, powered off). Speaking to the microphone in the ON state expands the voice and transmits it to the entire conference.

The system recognizes a position at which a participant raises his/her hand for remark. The system recognizes a direction designated by a moderator using a hand and the like.

Then, the system recognizes the raise-hand position of the participant and the direction designated by the moderator, and controls the microphone in a region in which they match to be turned ON.

In one embodiment of the system, the microphone of the person who does not input a voice to the microphone for a certain period of time is turned OFF. For example, when a plurality of participants raise their hands in the direction designated by the moderator, the microphones of the plurality of persons are turned ON, or determined as candidates to be turned ON. Meanwhile, when the microphone is configured to be turned OFF when the voice input is not performed for a certain period of time, the microphones other than that of the speaker is controlled to OFF even when, for example, a plurality of microphones are turned ON. In addition, the microphone is automatically turned OFF after the remark of the speaker ends.

In one embodiment of the system, the voice of the moderator is recognized, and the microphone to be turned ON is selected using the voice of the moderator. As described above, when there are a plurality of candidates whose microphones are turned ON, the microphone to be turned ON can be selected reflecting the intention of the moderator.

One embodiment of the system includes a heart rate sensor that measures a heart rate of the participant. Since the heart rate sensor is included, a remark of an abnormally exciting person can be obstructed. In addition, since a person trying to make remark becomes not a little nervous, using the heart rate sensor allows causing a person likely to make an appropriate remark to make a remark.

### EFFECTS OF INVENTION

One of the plurality of embodiments of this Description can provide a conference support system that can effectively determine a person intended by a moderator as a speaker by matching a raise-hand of a speaker with an indicating direction of the moderator and turning ON only a microphone of the matched speaker.

### BRIEF DESCRIPTION OF THE DRAWINGS

Fig. 1 is a flowchart according to one embodiment illustrating a process of determining a speaker by a conference support system.
Fig. 2 is a flowchart illustrating an exemplary speaker determination step.
Fig. 3 is a conceptual diagram illustrating an example of conference participants.
Fig. 4 is a conceptual diagram illustrating that a plurality of persons raise their hands at a request of a moderator and the moderator designates a speaker among the raise-hand participants using the moderator's arm.
Fig. 5 is a conceptual diagram illustrating a state where a computer obtains a region designated by the moderator.
Fig. 6 is a conceptual diagram illustrating an example in which a direction designated by the moderator is adjusted.

### DESCRIPTION OF PREFERRED EMBODIMENTS

The following describes embodiments of the present invention using the drawings. The present invention is not limited to the embodiments described below, but includes modifications of the following embodiments made by those skilled in the art within an obvious range as necessary.

A conference support system as one embodiment disclosed in this Description includes a plurality of voice input means, a plurality of ON/OFF control means, a raise-hand recognition means, a designated direction recognition means, and a speaker determination means.

The plurality of voice input means are means that receive voice input from participants. An example of the plurality of voice input means is a microphone. A conference support system preferably includes a voice outputting means that outputs the voice from the microphone. An example of the voice outputting means is a speaker, a pair of earphones, or a headset. The microphones may be installed on respective tables in a conference room. The microphone may be a portable microphone. When the microphones are installed on the tables in the conference room, the microphones only need to be each managed by an external device with ID information. When the microphones are portable, the microphones may be controlled by the ID information, and the microphones preferably each include a transmitting device, such as an antenna, and a location information transmitter, such as a GPS, so as to be each recognized by the external device.

The ON/OFF control means is means to control ON/OFF of each of the plurality of voice input means. The ON/OFF means, for example, a state where the power is on and a state where the power is off. In the microphone ON state, the voices of the participants can be collected and the voices can be input to the system. The voices are transmitted to the speaker and the like. Meanwhile, in the microphone OFF state, the microphone cannot collect the voices and remarks of the participants are not input.

When the microphone is portable, the microphone is preferably configured to receive a signal from outside and the ON/OFF of the microphone can be preferably controlled from outside. In this case, it is only necessary that the microphone includes an antenna to receive a wireless signal from outside and the ON/OFF of the microphone can be controlled based on the received wireless signal. The portable microphone only needs to be distributed to the participant at a reception and the like of the conference.

The raise-hand recognition means is means to recognize raise-hand positions of the participants corresponding to the respective plurality of voice input means.

One aspect of the raise-hand recognition means is a photographing means for photographing the participant or a sensor. An exemplary photographing means is a camera. An exemplary sensor is an infrared sensor. It is only necessary that, with a setting in which a height position corresponding to the raise-hand in the conference room can be photographed or sensed from a plurality of directions, the position of the raise-hand participant can be identified from photographing information or sensing information from the plurality of directions. For example, in a case of a quadrangular conference room, a camera or a plurality of infrared sensors are installed in a longitudinal direction, and a camera or a plurality of infrared sensors are installed in a lateral direction as well. Then, by analyzing the image or aggregating the sensing information, the position of the raise-hand participant can be identified.

One aspect of the raise-hand recognition means is the one including a transmitter. The transmitter worn on a dominant arm of the participant may have a wristband shape or a watch shape, or may have a finger ring shape. For example, the means may sense a move of the shoulder or the arm of the participant and transmit the sensing information from a transmitting unit, such as an antenna. Such means may be integrated with a microphone device. Then, the sensor measures the information based on the move of the shoulder or the move of the arm and transmits it from the antenna. An external device receives the sensing information (and ID information of the microphone) transmitted from the antenna, and determines whether the hand is raised or not. Thus, whether the hand is raised or not can be analyzed without being noticed by the participant. An exemplary sensor is a six-axis sensor, a (three-axis) gyro sensor, and a (three-axis) acceleration sensor. The six-axis sensor is a sensor that can detect an acceleration, a direction, and a rotation, and can further calculate a moving distance and a moving speed. Another exemplary sensor is a sensor that senses a pressure, a geomagnetic sensor, and a GPS (Global Positioning System) sensor. The transmitting unit is connected to the sensor so as to receive the sensing information from the sensor. Then, the transmitting unit transmits sensing data received from the sensor toward the external device. The sensing data may include acceleration data, angular speed data, and the like of the participant observed by the sensor corresponding to the sensor type.

The designated direction recognition means is means to recognize a direction designated by a specific person, such as a moderator. The designated direction recognition means preferably recognizes a position of the moderator or the like as well. Accordingly, the direction (or region) designated by the moderator can be recognized. For example, it is means that recognizes the direction of the arm when the moderator points the raise-hand participant with the arm. The designated direction recognition means may be achieved by a camera or an infrared sensor. The designated direction recognition means may be achieved by a sensor and a transmitter worn on a specific person, such as a moderator. The designated direction recognition means can be achieve similarly to the raise-hand recognition means.

The speaker determination means is means that turns ON the ON/OFF control means of the voice input means corresponding to the participant when the raise-hand position of the participant recognized by the raise-hand recognition means matches the direction designated by the specific person recognized by the designated direction recognition means.

The speaker determination means can be achieved by, for example, a computer. The computer includes an input unit, an output unit, a control unit, a calculation unit, and a storage unit. Then, a control program stored in the storage unit is read, and the control unit issues various commands. The control unit reads various kinds of data stored in the storage unit, and uses the read data and the data input from the input unit to cause the calculation unit to perform various operations. The data obtained through the arithmetic opexpressionerations by the calculation unit is stored in the storage unit as necessary, and output from the output unit. The input unit is a component to input various kinds of data to the computer. An exemplary input unit is a keyboard, an interface, and an antenna. A control program may be achieved by hardware, may be a computer-readable program stored in the storage unit, and may be a computer-readable program recorded in a recording medium, such as a CD-ROM.

The system recognizes a position at which the participant raises his/her hand for remark. The system recognizes a direction designated by the moderator using the hand and the like. Then, the system recognizes the raise-hand position of the participant and the direction designated by the moderator, and performs a control to turn ON the microphone in a region in which they match.

That is, this method includes a raise-hand recognition step of recognizing the raise-hand position of the participant,
a designated direction recognition step of recognizing the direction designated by the specific person, such as a moderator, and
a speaker determination step of turning ON the voice input means corresponding to the participant when the raise-hand position of the participant matches the direction designated by the specific person.

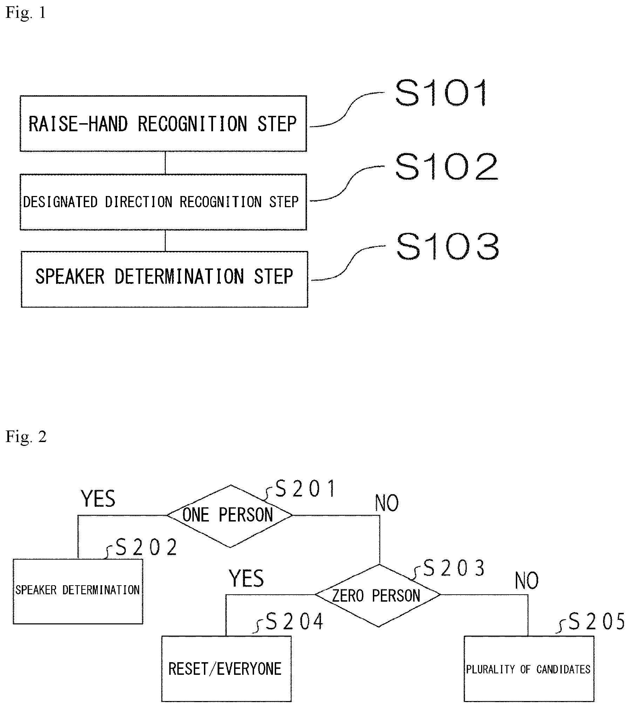

Fig. 1 is a flowchart according to one embodiment illustrating a process of determining a speaker by a conference support system.

### Raise-hand recognition Step (S101)

The raise-hand recognition means recognizes the raise-hand position of the participant. For example, since the microphone includes the various sensors and the antenna, displacement information of the shoulder or the arm sensed by the sensor is transmitted to the external device (computer) via the antenna. At this time, the microphone ID and the location information may be transmitted together. Then, the computer receives the information including the sensing information via the antenna and analyzes it, thereby allowing obtaining the location information of the participant corresponding to the microphone and whether the hand is raised or not. The raise-hand recognition step can be achieved as well by using a known method, such as a camera or an infrared sensor.

### Designated direction recognition Step (S102)

The designated direction recognition step is a step of recognizing the position and the designated direction of the moderator and the like.

The designated direction recognition means recognizes the position and the designated direction of the moderator and the like. The specific method is similar to that of the raise-hand recognition step.

### Speaker Determination Step (SI03)

This is a step of turning ON the voice input means corresponding to the participant when the raise-hand position of the participant matches the direction designated by the specific person.

As described above, the position of the raise-hand participant is recognized in the raise-hand recognition step, and the direction (region) designated by the specific person, such as a moderator, is recognized in the designated direction recognition step.

When a width is not provided to the direction designated by the moderator, the participant cannot be appropriately designated. Therefore, for recognizing the direction designated by the moderator, it is preferred that the computer performs a control such that a region having a certain amount of width allowed for the designated direction as a direction of an arm, a pointer, or the like from the position of the moderator or the moderator's arm as a starting point is set to the direction designated by the moderator. An exemplary angle for providing the width is 1° or more and 40° or less, may be 5° or more and 30° or less, or may be 5° or more and 20° or less. For example, when providing the width of 20°, it is only necessary to recognize a region of 10° from the starting point to each of the right and the left having the center in the actual designated direction as the designated direction.

Fig. 2 is a flowchart illustrating an exemplary speaker determination step.

In this example, it is determined whether the number of the raise-hand participant included in the direction (region) designated by the specific person is one or not (S201).

When the number of the raise-hand participants included in the direction (region) designated by the specific person is one (in the case of YES in S201), the participant is determined as the speaker, and the microphone corresponding to the participant is turned ON (S202).

When the number of the raise-hand participants included in the direction (region) designated by the specific person is not one (in the case of NO in S201), it is determined whether the number of the raise-hand participants included in the direction (region) designated by the specific person is zero or not (S203).

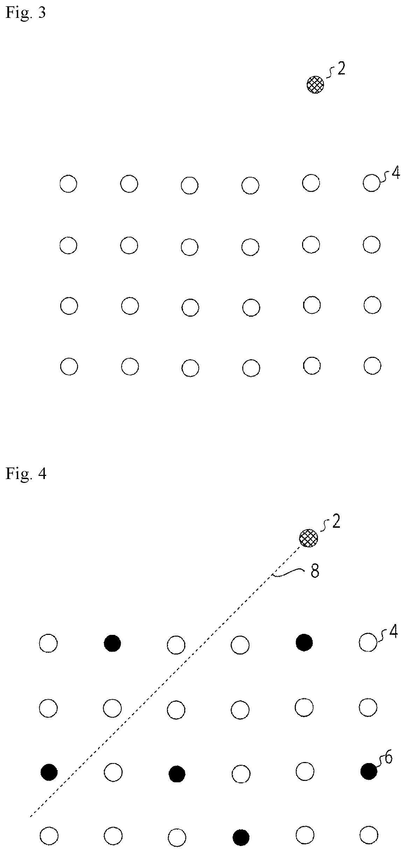

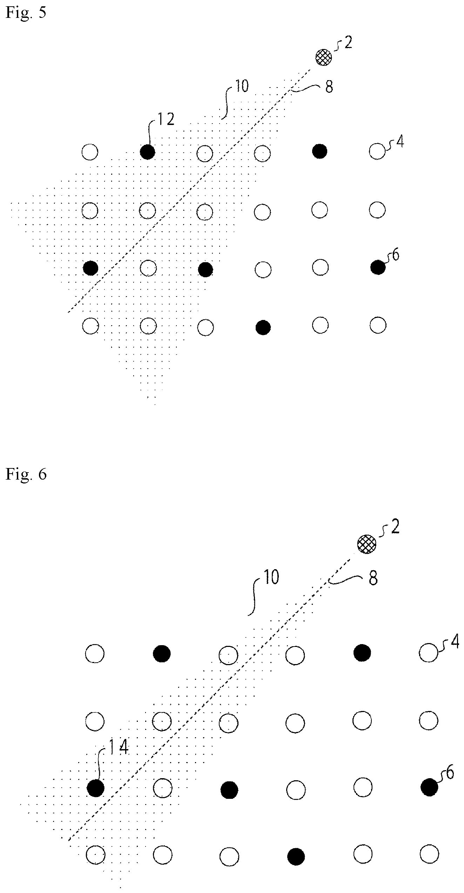

Fig. 3 is a conceptual diagram illustrating an example of conference participants. In this example, one moderator 2 and a plurality of participants 4 are present. When a conference starts, the moderator 2 requests the participants to raise their hands. Fig. 4 is a conceptual diagram illustrating that a plurality of persons raise their hands at the request of the moderator and the moderator designates a speaker among the raise-hand participants using the moderator's arm. In this example, to the raise-hand persons 6, the moderator indicates the one asking for a remark with the arm while saying, for example, "the one over there, please." In the diagram, a direction 8 designated by the moderator is illustrated by a dashed line. Fig. 5 is a conceptual diagram illustrating a state where a computer obtains the region designated by the moderator. The computer obtains the positions of the raise-hand participants 6. The computer also obtains the position of the moderator 2. Further, the computer obtains the direction designated by the moderator, and obtains a region 10 indicated by the direction designated by the moderator using the obtained direction. Then, in the case of Fig. 5, the region 10 as the direction designated by the moderator includes the three raise-hand persons. The persons included in the region 10 as the direction designated by the moderator among the raise-hand persons 6 can be referred to as speaker candidates 12. Fig. 6 is a conceptual diagram illustrating an example in which the direction designated by the moderator is adjusted. In this example, the speaker candidates 12 are narrowed to one person.

When the number of the raise-hand participants included in the direction (region) designated by the specific person is zero (in the case of YES in S203), the speaker is not determined (S204). In this case, for example, it is only necessary to determine the speaker by another algorithm. For example, the region (angle) provided with the width may be increased by a predetermined degree for each (for example, 1° for each) with respect to the actual indicating direction of the moderator, and the processing steps may be repeated from Step 201. When the speaker cannot be identified by this process, the microphones of all the participants may be turned ON. Furthermore, the speaker may be determined using any process described below.

When the number of the raise-hand participants included in the direction (region) designated by the specific person is not zero (in the case of NO in S203), a plurality of participants are determined as the speaker candidates (S205). In this case, the microphones corresponding to all of the plurality of persons may be turned ON, and furthermore, the speaker candidates may be decreased in number to determine the speaker using another algorithm.

Another embodiment disclosed in this Description is a conference support system that further includes timekeeping means 13 and OFF control means 15. The timekeeping means 13 measures a time period. The OFF control means 15 controls a voice input means to OFF using an ON/OFF control means corresponding to the voice input means when a voice input to the voice input means determined by a speaker determination means is not performed for a certain period of time. The timekeeping means 13 is means ordinarily included in a computer.

In this embodiment, the microphone of the person who does not input a voice to the microphone for a certain period of time is turned OFF. For example, when a plurality of participants raise their hands in the direction designated by the moderator (in the case of S205), the microphones of the plurality of persons only needs to be turned ON once. Then, it is only necessary to turn OFF the microphone of the person who does not input a voice to the microphone for a certain period of time.
Accordingly, even when a plurality of microphones are turned ON, the microphones other than that of the speaker are controlled to OFF. The certain period of time only needs to be measured by the timekeeping means 13. For example, it is only necessary that after determining the speaker candidates in S205, the input from the microphones of the respective candidates are measured, and when the voice input of a certain level or more (threshold or more) is not performed for the certain period of time measured by the timekeeping means 13, the participant corresponding to the microphone is determined not to be the speaker, thus turning the microphone OFF. When the timekeeping means 13 is included, it is only necessary to control the microphone to be automatically turned OFF when the voice input of the certain level or more (threshold or more) is not performed for the certain period of time after the remark of the speaker ends.

Another embodiment disclosed in this Description further includes moderator voice input means 17, moderator voice recognition means 19, and moderator voice analysis means 21. The moderator voice input means 17 receives the voice input of the specific person. The moderator voice recognition means 19 recognizes the voice of the specific person received by the moderator voice input means 17. The moderator voice analysis means 21 analyzes the voice of the specific person recognized by the moderator voice recognition means 19. The speaker determination means determines the voice input means to be turned ON considering the voice of the specific person analyzed by the moderator voice analysis means 21.

For example, Japanese Patent No. 4551105 discloses a conference support system using a voice recognition. The conference support system using the voice recognition is publicly known. That is, the moderator voice input means 17, the moderator voice recognition means 19, and the moderator voice analysis means 21 can be achieved using the known technique. For example, when the microphone is installed on the table, or when the microphone is a type of transmitting location information (for example, portable microphone), a computer as an external device obtains the microphone position. Then, for example, when the moderator makes a remark of a person in the "rear" of a "right row," "please," the voice recognition is performed, and the "rear" in the "right row" is identified. Using the information allows further narrowing the region designated by the moderator. That is, the participants in the front are excluded from the speaker even when they have been determined as the speaker candidates.

When the system includes a camera (the camera may be a part of the raise-hand recognition means), the system can recognize an image of the participant. For example, when the moderator makes a remark of a person wearing a "blue tie," "please," the participant wearing the blue tie recognized by the camera is selected from the speaker candidates and determined as the speaker.

Another embodiment disclosed in this Description is a conference support system that further includes a plurality of heart rate sensors 23a, 23b, and 23c that measure heart rates of the participants corresponding to the respective plurality of voice input means, and determines the voice input means to be turned ON considering the measurement values of the heart rates measured by the plurality of heart rate sensors 23a, 23b, and 23c.

The heart rate sensor is the known one included in what is called a sport watch. Using the heart rate sensor allows measuring the heart rate of a target person in real-time. The heart rate sensor is preferably configured to transmit information on the heart rate (for example, together with information on the microphone) by a transmitter.

The computer receives the information on the heart rate measured by the heart rate sensor. Then, the computer stores the heart rate in the storage unit. The computer stores heart rate variation patterns and information on psychological states of the users corresponding to the respective variation patterns. Then, when determining that the heart rate of one person varies, the computer reads the variation pattern to obtain the psychological state. For example, when there are a plurality of speaker candidates, the computer reads the information on the heart rates of the candidates, and performs a psychological analysis for each candidate using the variation patterns. Since a person designated by the moderator among the plurality of candidates recognizes that he/she himself/herself is selected, the person becomes nervous different from the other candidates. The nervous state causes a specific heart rate variation. Therefore, measuring the heart rate of the participant using the heart rate sensor allows the appropriate determination of the speaker.

### INDUSTRIAL APPLICABILITY

The invention can be used as the conference support system in the information industry and the office equipment industry.

### DESCRIPTION OF REFERENCE SIGNS

- 2: Moderator
- 4: Participant
- 6: Raise-hand person
- 8: Actual indicating direction of moderator
- 10: Indicating direction (region) of moderator
- 12: Speaker candidate

## Claims

1. A conference support system comprising:
a plurality of voice input means that receive voice input;
a plurality of ON/OFF control means that control ON/OFF of each of the plurality of voice input means, the plurality of ON/OFF control means corresponding to the respective plurality of voice input means;
raise-hand recognition means that recognizes raise-hand positions of participants corresponding to the respective plurality of voice input means;
designated direction recognition means that recognizes a direction designated by a specific person; and
speaker determination means that turns ON the ON/OFF control means of the voice input means corresponding to a participant recognized by the raise-hand recognition means when the raise-hand position of the participant matches the direction designated by the specific person recognized by the designated direction recognition means.

2. The conference support system according to claim 1, further comprising:
timekeeping means that measures a time period; and
OFF control means that controls the voice input means to OFF using the ON/OFF control means corresponding to the voice input means when a voice input to the voice input means determined by the speaker determination means is not performed for a certain period of time.

3. The conference support system according to claim 1, further comprising:
moderator voice input means that receives a voice input of the specific person;
moderator voice recognition means that recognizes the voice of the specific person received by the moderator voice input means; and
moderator voice analysis means that analyzes the voice of the specific person recognized by the moderator voice recognition means, wherein
the speaker determination means determines the voice input means to be turned ON considering the voice of the specific person analyzed by the moderator voice analysis means.

4. The conference support system according to claim 1, further comprising
a plurality of heart rate sensors that measure heart rates of the participants corresponding to the respective plurality of voice input means, wherein
the voice input means to be turned ON is determined considering measurement values of the heart rates measured by the plurality of heart rate sensors.
